Europäisches Patentamt

European Patent Office

Office européen des brevets

(19)

(11) EP 1 184 665 A1

(12) EUROPEAN PATENT APPLICATION
published in accordance with Art. 158(3) EPC

(43) Date of publication:
06.03.2002 Bulletin 2002/10

(51) Int Cl.⁷: **G01N 33/53**, G01N 33/573,
C07K 16/40, C12Q 1/48,
C12N 15/54, C12N 9/12

(21) Application number: 00929789.6

(22) Date of filing: 18.05.2000

(86) International application number:
**PCT/JP00/03193**

(87) International publication number:
**WO 00/72011 (30.11.2000 Gazette 2000/48)**

(84) Designated Contracting States:
**AT BE CH CY DE DK ES FI FR GB GR IE IT LI LU
MC NL PT SE**

(30) Priority: **21.05.1999 JP 14118799**

(71) Applicant: **Medical & Biological Laboratories Co.,
Ltd.**
**Nagoya-shi, Aichi 460-0002 (JP)**

(72) Inventors:
• **OGAWA, Akira**
**GUNMA 370-1202 (JP)**

• **KOBAYASHI, Toshiko,**
**Medical & Biol. Lab. Co., Ltd.**
**Ina-shi, Nagano 396-0002 (JP)**
• **YANO, Minoru**
**San Diego, CA 92108 (US)**
• **TAMAI, Katsuyuki, Medical & Biol. Lab. Co., Ltd.**
**Ina-shi, Nagano 396-0002 (JP)**

(74) Representative: **VOSSIUS & PARTNER**
**Siebertstrasse 4**
**81675 München (DE)**

(54) **METHOD FOR MEASURING PROTEIN KINASE ACTIVITY**

(57)    The present invention provides a method for evaluating the phosphorylation of a substrate peptide phosphorylated by DNA-dependent protein kinase (DNA-PK), ATM, and ATR, and a method for measuring protein kinase (or protein phosphatase) activity towards the substrate peptide. By applying the assay method of the present invention, screening of compounds that regulate activity of these enzymes can be accomplished.

EP 1 184 665 A1

**Description**

Technical Field

The present invention relates to a method for measuring protein kinase activity or protein phosphatase activity using an antibody that identifies a phosphorylated state, a method of screening for an inhibitor or an promoter of a protein kinase or protein phosphatase, and a kit for these assay and screening methods.

Background art

DNA-dependent protein kinase (DNA-PK), ATM (AT mutated) and ATR (ATM and rad3 related), are all serine/ threonine protein kinases related to the cell cycle through phosphorylation of p53, which is an expression product of a tumor suppressor gene.

When cells proliferate, a periodically repeated process occurs in which the genomic DNA of the cells are replicated and distributed evenly to daughter cells, after which the cells divide. Such a cycle is called the cell cycle. The cell cycle can be divided into G1 phase (DNA synthesis preparation period), S phase (DNA synthesizing period), G2 phase (meiosis preparation period), and M phase (mitotic phase). Cells divide by sequentially undergoing each stage.

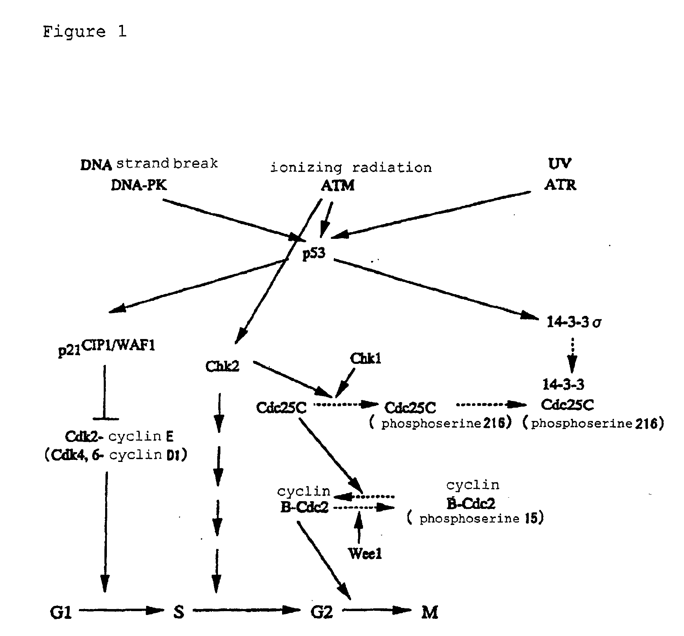

When DNA is damaged by ultraviolet irradiation, ionizing irradiation, drugs, and such, mammalian cells arrest their cell cycle normally at G1 phase, S phase or G2/M phase to repair the damage. This phenomenon is called the checkpoint mechanism, and the signal transduction pathway can be illustrated as in Figure 1. In response to DNA damage, p53 is activated and induces expression of p21CIP1/WAF1 and 14-3-3σ (S.J. Elledge. Science Vol 274, p1664, 1996; Paul Nurse. Cell Vol 91, p865, 1997). p21CIP1/WAF1 binds to Cyclin E-Cdk2, Cyclin D-Cdk 4 and 6, which are necessary for the progression of G1 phase, and inhibits their protein kinase activities (S.J. Elledge. Science Vol 274, 1996; D.O. Morgan. Nature Vol 374, p131, 1995). G1 arrest is accomplished by this mechanism.

On the other hand, 14-3-3σ inactivates Cdc25C by binding to phosphorylated Cdc25C (phosphoserine 216), which is phosphorylated by Chk1 or Chk2, and promoting nuclear localization (Paul Nurse. Cell Vol 91, p865, 1997). Active Cyclin B-Cdc2 complex necessary for the progression of G2/M phase is inactivated by phosphorylation with Weel protein kinase, and inactive Cyclin B-Cdc2 (phosphotyrosine 15) is activated by dephosphorylation with Cdc25C protein phosphatase (Paul Nurse. Cell Vol 91, p865, 1997). Therefore, G2 arrest is accomplished by the induction of 14-3-3σ protein expression. Moreover, S arrest is accomplished through Chk2 protein kinase that is activated by DNA damage (S. Matsuoka et al. Science Vol 282, p1893, 1998).

ATM (AT mutated), which is the gene responsible for AT (Ataxia telangiectasia) known as a human genetic disease, is activated when cells receive ionizing irradiation, and phosphorylates Ser15 of p53 (S. Banin et al. Science Vol 281, p1674, 1998; C.E. Canman et al. Science Vol 281, p1677, 1998). Moreover, ATR (ATM and rad3 related) activated by ultraviolet irradiation of cells also phosphorylates Ser15 of p53 (C.E. Canman et al. Science Vol 281, p1677, 1998). DNA-PK activated by DNA double-strand break phosphorylates Ser15 and Ser37 of p53 (S.P. LEES-MILLER et al. Mol. Cell. Biol. Vol 12, p5041, 1992). The phosphorylation of the 15th serine residue of the tumor suppressor gene p53 by serine/threonine protein kinases DNA-PK, ATM, and ATR, causes the dissociation of Mdm2 from p53 (S. Shieh et al. Cell Vol 91, p325, 1997). As a result of the obstruction of degradation by the Mdm2 protein-mediated ubiquitin-proteasome system, the half-life of p53 protein is lengthened by the phosphorylation of the 15th serine residue, and the p53-mediated checkpoint signal that follows DNA damage such as above is transmitted.

Cancer therapy includes, radiotherapy, chemotherapy, and immunotherapy. In radiotherapy, it is presumed that selective killing of cancer cells would become possible if a drug that blocks or decreases the function of DNA damage checkpoints of cancer cells existed. Moreover, a drug that increases checkpoint functions in normal cells is also considered to be effective for radiation therapy. In AT patients, it is known that sensitivity to ionizing irradiation is 3-4 times higher than in healthy individuals (C.H. Westphal et al., Nat. Genet. Vol. 16, p397, 1997). In cells derived from AT patients, G1 phase, S phase or G2/M phase arrest following ionizing irradiation is not observed (K. Savitsky et al. Science Vol. 268, p1749, 1995, W.A. Cliby et al. EMBO J. Vol 17, p159, 1998). However, AT cells are not highly sensitive to ultraviolet irradiation (W. A. Cliby et al. EMBO J. Vol. 17, p159, 1998). These facts suggest that an inhibitor truly specific to ATM would become a drug that remarkably improves the sensitivity of the cells toward ionizing radiation, without influencing ultraviolet irradiation checkpoints. Low-dose radiation therapy is also expected to become possible if there is a truly specific inhibitor for ATM.

A system for measuring phosphorylation activity will be useful not only for screening drugs against ATM, but also for developing drugs against ATR and DNA-PK. It is known that protein kinases such as DNA-PK, ATR or ATM phosphorylate other proteins such as c-Abl, PHAS-I, IκBα, RPA, and c-Jun, as well as p53. c-Abl is one of the oncogenes activated in 90% or more of chronic myelocytic leukemia cases. PHAS-I (phosphorylated heat and acid stable protein regulated by insulin) is a translational regulator that is a substrate of MAPK. IκBα is a protein involved in regulating the activity of the transcriptional regulator, NF-κB. RPA (replication protein A) is a single-stranded DNA binding protein being involved in the restoration and recombination of DNA. c-Jun is an intranuclear oncogene that is a constituent of the transcription factor AP1. Therefore, a simple and easy assay principle by which the protein kinase activity of the ATM, ATR or DNA-PK can be understood has an important meaning in research for elucidating the cell cycle.

However, up to now, the measurement of protein kinase activity required special equipment, since it used γ-$^{32}$P-ATP labeled with $^{32}$P.

Moreover, since DNA-PK, ATM, and ATR are serine/threonine protein kinases with similar primary structures belonging to the PI3K family, it was not possible to measure these enzyme activities separately. Thus, the development of a simple and easy protein kinase activity assay system was required.

On the other hand, a simple and easy protein kinase activity assay method for measuring PKA and PKC activity has been put to practical use. This method measures the protein kinase activity by detecting enzymic phosphorylation of a synthetic peptide called the PS peptide by using an antibody recognizing the phosphorylated PS peptide (T. Yano et al. Peptide Chemistry A. Suzuki (Ed) : 293-298,1991). However, the PS peptide, which is the substrate peptide in this method, cannot be a substrate for DNA-PK, ATM or ATR, which are protein kinases of p53. In addition, the antibody used in this method for detecting the level of phosphorylation cannot be used to measure the protein kinase activity of p53 since it does not recognize phosphorylated p53. Antibodies that recognize phosphorylated p53 are well known (C.E. Canman et al. Science Vol. 281, 1677, 1998), but it is not known that the protein kinase activity of p53 can be assayed by using these antibodies.

Disclosure of the Invention

An objective of the present invention is to provide a method for measuring the activity of DNA-PK, ATM and ATR, which phosphorylate p53, and the like, and a method for measuring protein phosphatase activity against p53, or the like, which are phosphorylated by the action of these protein kinases. Moreover, an objective of the present invention is to provide a method for screening a compound that regulates the protein kinase activity of DNA-PK, ATM, and ATR. In addition, an objective of the present invention is to provide an assay method in which not simply the activities of these protein kinases are measured, but activities of several protein kinases existing in the same sample can also be individually monitored. Additionally, an objective of the present invention is to provide a kit for these assay and screening methods.

To solve the above-mentioned issues, the present inventors thought that it may be possible to monitor protein kinase activities by immunologically measuring the changes of phosphorylation levels of a substrate peptide that is phosphorylated by DNA-PK, ATM and ATR. The present inventors focused their attention on the fact that SQ motif of p53 or the like, or serine residue comprised in the SQE motif, can be the target of phosphorylation by these protein kinases and the target of dephosphorylation, and zealous research was conducted. As a result, it was found that the phosphorylation level of the substrate peptide can be evaluated simply and easily by using an antibody against phosphorylated p53. In addition, by using the antibody, the present inventors found that it is possible to screen a compound that inhibits or promotes the phosphorylation or dephosphorylation of the substrate peptide by an enzyme action, and thus, the present invention was completed.

Namely, the present invention relates to the following method for measuring protein kinase activity, method for measuring protein phosphatase activity, and method of screening for a compound that regulates activities of protein kinases and protein phosphatases based on this assay method. Namely, the present invention relates to:

(1) a method for measuring protein kinase activity of a protein kinase in a sample, wherein the protein kinase is selected from the group consisting of DNA-PK, ATM, and ATR, and said method comprises the following steps of,

(a) contacting the sample with a substrate peptide phosphorylated by the protein kinase under conditions necessary for the phosphorylation reaction, and,
(b) detecting a change in the phosphorylation level of the substrate peptide based on a change in reactivity of the substrate peptide with an antibody that identifies the phosphorylation state of the substrate peptide;

(2) a method of screening for a compound that inhibits or promotes the protein kinase activity of a protein kinase, wherein the protein kinase is selected from the group consisting of DNA-PK, ATM, and ATR, and wherein the method comprises the following steps of,

(a) contacting the protein kinase selected from the group consisting of DNA-PK, ATM, and ATR and a substrate peptide phosphorylated by the protein kinase and incubating the protein kinase and the substrate peptide under conditions necessary for the phosphorylation reaction in the presence of a test compound,

(b) detecting a change in phosphorylation level of the substrate peptide based on a change in reactivity of the substrate peptide with an antibody that identifies the phosphorylation state of the substrate peptide, and,
(c) selecting a compound that decreases or increases the change in the phosphorylation level of the substrate peptide by comparing with the phosphorylation level of the substrate peptide in the absence of the test compound;

(3) a method for measuring protein phosphatase activity of a protein phosphatase against a phosphorylated protein in a sample, wherein the protein is phosphorylated by a protein kinase selected from the group consisting of DNA-PK, ATM, and ATR, and wherein the method comprises the following steps of,

(a) contacting a sample with a substrate peptide comprising at least the phosphorylation site of the phosphorylated protein and incubating the substrate peptide and the sample under conditions necessary for the dephosphorylation reaction, and,
(b) detecting a change in the phosphorylation level of the substrate peptide based on a change in reactivity of the substrate peptide with an antibody that identifies the phosphorylation state of the substrate peptide;

(4) a method of screening for a compound that inhibits or promotes the protein phosphatase activity of a protein phosphatase against a phosphorylated protein, wherein the protein is phosphorylated by a protein kinase selected from the group consisting of DNA-PK, ATM, and ATR, and wherein the method comprises the following steps of,

(a) contacting a protein phosphatase against the phosphorylated protein and a substrate peptide comprising at least a phosphorylation site of phosphorylated protein, and incubating the protein phosphatase and the substrate peptide under conditions necessary for the dephosphorylation reaction, in the presence of a test compound,
(b) detecting changes in dephosphorylation level of a substrate peptide based on a change in reactivity of the substrate peptide with an antibody that identifies the phosphorylation state of the substrate peptide, and,
(c) selecting a compound that decreases or increases changes in the dephosphorylation level of a substrate peptide by comparing with the dephosphorylation level of the substrate peptide in the absence of the test compound;

(5) a method according to any one of (1) to (4), wherein the substrate peptide comprises an amino acid sequence comprising the SQ motif or the SQE motif;
(6) the method according to (1), wherein the method separately measures protein kinase activities of several protein kinases coexisting in a sample, wherein each protein kinase phosphorylates a different site of the substrate peptide, and wherein the antibody that identifies the phosphorylation state identifies a phosphorylation site;
(7) the method according to (6), wherein the protein kinase is ATM or ATR, and the antibody is an antibody that identifies the phosphorylation state of the SQE motif comprising 15th serine of p53;
(8) the method according to (6), wherein the protein kinase is DNA-PK, and the antibody is an antibody that identifies the phosphorylation state of the SQ motif comprising serine 37 of p53;
(9) a method for measuring DNA-PK activity, wherein the method comprises the following steps of,

(a) measuring the protein kinase activity in a sample by the method according to (1) in the absence of double-stranded DNA,
(b) measuring the protein kinase activity in a sample by the method according to (1) in the presence of double-stranded DNA, and,
(c) determining the protein kinase activity derived from DNA-PK by comparing results of (a) and (b);

(10) a method according to any one of (1) to (4), wherein the reactivity of the antibody towards a phosphorylated substrate peptide is higher than the reactivity towards an unphosphorylated substrate peptide;
(11) a method according to any one of (1) to (4), wherein the reactivity of the antibody towards an unphosphorylated substrate peptide is higher than the reactivity towards a phosphorylated substrate peptide;
(12) a method according to any one of (1) to (4), wherein the substrate peptide is labeled;
(13) a method according to any one of (1) to (4), wherein the substrate is solid-phased;
(14) a method according to any one of (1) to (4), wherein the antibody is labeled;
(15) a method according to any one of (1) to (4), wherein the phosphorylation level of the substrate peptide is evaluated by ELISA;
(16) a compound that regulates the activity of a protein kinase selected from the group consisting of DNA-PK, ATM, and ATR, wherein the compound is isolated by the method of screening according to (2) ;

(17) a compound that regulates the protein phosphatase activity of a protein phosphatase towards a protein phosphorylated by a protein kinase selected from the group consisting of DNA-PK, ATM, and ATR, wherein the compound is isolated by the method of screening according to (4) ;

(18) a compound according to (16) or (17), wherein the compound is natural;

(19) an antibody for use in a method according to any one of (1) to (4), wherein the method identifies the phosphorylation state of a substrate peptide; and,

(20) a kit for a method according to any one of (1) to (4), comprising an antibody that identifies the phosphorylation state of a substrate peptide.

In the present invention, "peptide" indicates a compound comprising several amino acids bound together by a peptide bond, and is not restricted by chain length. Therefore, proteins are also included in "peptide" of the present invention. The subject of the measurement of the present invention, protein kinase, which is the subject of the measurement of the present invention, is selected from the group consisting of DNA-PK, ATM, and ATR. Samples used in the measurement can be, in addition to natural enzymes derived from human tissues, recombinant enzymes obtained by expressing the genes encoding them using suitable expression systems. Moreover, the origin of the enzyme is not limited to humans, and can be a homologue obtained from other vertebrates and microorganisms. In addition, not only enzymes comprising the same amino acid sequence as the natural enzyme protein, but also any enzyme protein with essentially the same enzyme activity, such as mutant proteins comprising only domains which support the expression of enzyme activity (protein kinase domain), or fusion proteins in which this region is fused with another protein, can be the subject for measuring protein kinase activity by the present invention.

The present invention relates primarily to a method for measuring protein kinase activity, in which an antibody that binds specifically to a substrate peptide phosphorylated by DNA-PK, ATM, and ATR is used. More specifically, the present invention relates to a method comprising the following steps of:

(a) contacting the sample with a substrate peptide phosphorylated by the protein kinase under conditions necessary for the phosphorylation reaction, and,

(b) detecting a change in the phosphorylation level of the substrate peptide based on a change in reactivity of the substrate peptide with an antibody that identifies the phosphorylation state of the substrate peptide;

Substrate peptide used in the present invention is not especially limited, as long as it is phosphorylated by DNA-PK, ATM, and ATR. Specifically, it is known that a peptide comprising the SQ motif comprising, for example, the following amino acid sequences, has a serine (S) residue that is phosphorylated by these protein kinases.

SQ motif:

$$X_{-n} \ldots X_{-4} \, X_{-3} \, X_{-2} \, X_{-1} \, S \, Q \, X_{+2} \, X_{+3} \, X_{+4} \ldots X_{+n}$$

Herein, when the serine residue at the phosphorylation site is designated as 0, an arbitrary amino acid residue X that is n amino acid residues from 0 towards the amino terminal is written as $X_{-n}$, an arbitrary amino acid residue X that is n amino acid residues from 0 towards the carboxyl terminal is written as $X_{+n}$. Peptides comprising this motif are assumed to be phosphorylated in general at the position of S. Since the Km value of the protein kinase changes according to the amino acid of X, if a substrate peptide comprising an amino acid sequence with a lower Km value is used, protein kinase assay can be conducted with a higher sensitivity. For instance, in the case of ATM and ATR, it is presumed that phosphorylation easily occurs especially when the position $X_{+2}$ is E in the SQ motif. Such an amino acid sequence, which is called the SQE motif, becomes a preferable substrate peptide when aiming at measuring activities of ATM and ATR.

SQE motif:

$$X_{-n} \ldots X_{-4} \, X_{-3} \, X_{-2} \, X_{-1} \, S \, Q \, E \, X_{+3} \, X_{+4} \, X_{+5} \ldots X_{+n}$$

In addition, the following proteins can be given as examples of proteins derived from the living body that can be used as substrate peptides.

p53 (L.J. Ko and C. Prives. Genes and Dev. Vol 10, p1054, 1996; A.L. Levine. Cell Vol.88, p323, 1997)

c-Abl (S. Kharbanda et al. Science Vol 386, p732, 1997)

PHAS-I (C. Hu et al. Proc. Natl. Acad. Sci. USA Vol 91, p3730, 1994; G.J. Brunn et al. Science Vol 277, p99, 1997; S. Banin et al. Science Vol 281, p1674, 1998)

IκBα (A.A. Beg and A.S. Baldwin Jr. Genes & Dev. Vol 7, p2064)

RPA (R.G. Shao et al. EMBO J. Vol 18, p1397, 1999)

c-Jun (T. Smeal et al. Mol. Cell. Biol. Vol 12, p3507, 1992)

The substrate peptide can be a natural peptide, a recombinant peptide prepared by genetic engineering, or a synthetic peptide. It can be fused with another peptide (for instance, glutathione S-transferase) to facilitate purification of the peptide. The substrate peptide is used in an amount sufficient for the expected enzyme activity. For instance, when the phosphorylation reaction is conducted at a certain substrate peptide concentration and most of the substrate peptide is phosphorylated, there is a concern that a shortage in the substrate peptide may occur. In such a case, increasing of the relative concentration of substrate peptide by, for example, increasing substrate concentration or diluting samples is desired.

In the present invention, the substrate peptide is contacted with the sample under conditions needed for the phosphorylation by the above-mentioned protein kinase. "Conditions needed for the phosphorylation" means incubating in reaction media which give a pH necessary for the maintenance of subject protein kinase activity, in the presence of a component necessary for the reaction along with the substrate peptide, at the proper reaction temperature. As a component necessary for the reaction, a substrate providing a phosphate group necessary for the phosphorylation reaction, a protein phosphatase inhibitor added for the purpose of protecting the phosphorylation product can be given. As substrates providing phosphate groups, adenosine 5'-triphosphoric acid (ATP) and the like are used in general, and β-glycerophosphoric acid and the like are used as protein phosphatase inhibitors. Besides this, in the case of measuring protein kinase activity of DNA-PK, fragmented double-strand DNA is exemplified, and in the case of measuring protein kinase activity of ATM or ATR, Mn ion is exemplified as the component other than substrate peptide. A reaction buffer used in the present invention for ATM/ATR that consists of the following composition can be given as a buffer for the phosphorylation reaction.

Reaction buffer for ATM/ATR:

10 mM HEPES (pH 7.5)
50 mM β-glycerophosphoric acid
50 mM NaCl, 10 mM $MgCl_2$
10 mM $MnCl_2$
5 mM ATP
1 mM DTT

DNA-PK reaction buffer:

25 mM HEPES pH 7.5
75 mM KCl
10 mM $MgCl_2$
1 mM DTT
0.2 mM EGTA
0.25 mM ATP
10 μg/ml ultrasonicated double-strand DNA
50 mM β-glycerophosphoric acid

Under preferable incubation conditions, when a protein kinase against a substrate peptide is present in the sample, the substrate peptide is phosphorylated according to the protein kinase activity. Here, specific activity assay methods can be constructed for the respective enzymes by taking advantage of the fact that a specific component supports the phosphorylation reaction of each enzyme. For instance, since DNA-PK requires a double-stranded DNA fragment for the expression of activity (T. Carter et al., Mol. Cel. Biol. Vol 10, p 6460-6471 (1990)), protein kinase activity derived from DNA-PK can be determined based on the difference of protein kinase activity in the presence or absence of double-stranded DNA in the reaction mixture.

In the present invention, the phosphorylated level of a phosphorylated substrate peptide is evaluated by an immunological reaction with an antibody that can identify a phosphorylation state of the substrate peptide. In the present invention, the level of phosphorylation is used as a term indicating not only the ratio of the phosphorylated peptide in the substrate peptide, but also the degree of phosphorylation on the substrate peptide molecule. Therefore, the increase of the phosphorylation level of the substrate peptide means both the state in which the ratio of phosphorylated peptide is increased, and the state in which the number of phosphorylated sites on the substrate peptide is increased.

On the other hand, the antibody used in the present invention that can identify a phosphorylation state means an antibody whose reactivity with the substrate peptide changes in the presence or absence of phosphorylation. Specifically, antibodies having a higher (or lower) reactivity to a phosphorylated substrate peptide rather than to an unphosphorylated substrate peptide can be exemplified. For instance, an antibody that specifically recognizes p53 phosphorylated by DNA-PK is well-known (S-Y. Shieh et al. Cell Vol 91, p325, 1997). Similar antibodies can be prepared by using a method well-known to one skilled in the art (for instance, Cell Technology, separate volume, anti-peptide antibody experiment protocol, 1994, Shujunsha; B. M. Turner and G. Fellows, Eur. J. Biochem. 179, 131-139, 1989; S. Muller etal. Molecular Immunology Vol 24, 779-789, 1987; Pfeffer, U. , Ferrari, N. and Vidali, G.J. Bio. Chem. 261, 2496-2498, 1986). The antibody may be a monoclonal antibody, or it may be polyclonal antibody.

For instance, polyclonal antibodies specific to a phosphorylated peptide can be purified from antiserum obtained by immunizing an animal with the phosphorylated peptide, using an unphosphorylated peptide-immobilized column (non-specific antibody absorption column) or phosphorylated peptide-immobilized column (specific antibody purification column). Alternatively, hybridoma producing a specific antibody can be cloned from hybridoma established from antibody producing cells of animals immunized with the phosphorylated peptide, by screening for a hybridoma producing the specific antibody. Screening is conducted by examining the reactivity of the culture supernatant to unphosphorylated and phosphorylated peptide by the ELISA method, and selecting clones with a high reactivity against the phosphorylated peptide. By culturing the hybridoma cloned, the desired monoclonal antibody can be purified from the culture supernatant or ascites.

In a preferable embodiment of the present invention, an assay method that not only identifies a phosphorylation state but also distinguishes structural differences including the amino acid sequence comprising the substrate peptide is provided. Protein kinases do not always phosphorylate the same site of a substrate peptide. For instance, when DNA-PK phosphorylates p53, the phosphorylation progresses mainly on two serine residues, Ser15 and Ser37. On the other hand, when ATM and ATR phosphorylate the same p53, the main phosphorylation site is the Ser15 serine residue. Therefore, by using antibodies that recognize the amino acid sequence including serine residues that are phosphorylated, phosphorylation by DNA-PK and that by ATM or ATR can be separately evaluated. Namely, the present invention provides an assay method by which individual protein kinase activities can be separately determined using a sample in which several protein kinases may coexist.

The reaction of the substrate peptide and the antibody can be detected by using a well-known immunoassay principle. In general, the separation of unreacted components can be easily achieved by using either the substrate peptide or the antibody in a solid phase. The method of making the substrate peptide or the antibody a solid phase is well-known. For instance, a substrate peptide or an antibody can be directly immobilized on to a solid phase by a chemical bond or physical adsorption. By biotinizing the substrate peptide, it can also be indirectly solid-phased by capturing it on to the solid phase sensitized with streptavidin. In general, when the substrate peptide is solid-phased, it is conducted under conditions in which the adsorption of the protein to the carrier is saturated. In this case, solid-phasing of the biotinated substrate peptide can be conducted at any timing such as before or after contacting with the sample, or even simultaneously to the contact. In addition, systems other than the avidin-biotin system can be applied to the present invention as long as it is a combination having mutual affinity. For instance, the substrate peptide can be prepared as a conjugate with an appropriate antigenic material and captured using a solid phase sensitized with an antibody against the antigenic material.

In the present invention, a solid phase used in general such as an inner wall of a reaction container, a granular carrier, or bead-shaped carrier, can be used as the solid phase for immobilizing the substrate peptide or antibody. As for the material, a material generally used for physically adsorbing or chemically binding proteins in general can be used. Specifically, a microtiter plate made of polystyrene, or the like, is used.

In the present invention, a simple and easy operation can be achieved by labeling either the substrate peptide or the antibody, when evaluating the changes of phosphorylation level based on an immunoassay principle. The label is not especially limited as long as it has a sensitivity sufficient enough for detection. For example, enzyme labels such as peroxidase, β-D-galactosidase, alkaline protein phosphatase, glucose-6-phosphate dehydrogenase, and acetylcholinesterase, fluorescent labels such as delphinium, and radiolabels can be used. Moreover, not only direct labeling, but also the so-called indirect labeling system can be used, in which a material that binds specifically to the antibody, for example a secondary antibody, protein A, protein G, protein A/G (fusion protein of protein A and G), and such are labeled.

The phosphorylation level of the substrate peptide can be evaluated by a method well-known to one skilled in the art (for instance, refer to, "Super-high sensitivity enzyme immunoassay method" Ishikawa E. , Gakkai-Syuppan center (1993)). Namely, when using the combination of an antibody reacting to a phosphorylated peptide and a labeled substrate peptide, the amplitude of the signal originating from the labeled substrate peptide that reacted with the antibody is proportional to protein kinase activity. In contrast, when the antibody reactive to an unphosphorylated substrate peptide is used, the amplitude of the signal is inversely proportional to phosphorylation activity. Phosphorylation levels can be quantified based on a calibration curve, which is made beforehand using a substrate peptide with a known phosphorylation level as the standard sample. Alternatively, a qualitative comparison can be conducted using a sample free of protein kinase activity as the control.

Any sample suspected to have DNA-PK, ATM and ATR can be the target of the method for measuring protein kinase activity of the present invention. As typical samples, tissue samples derived from humans and non-human animals, extracts of various cultured cells, culture supernatants of cells, blood, urine, body fluids, sweat, saliva, body secretions such as milk, and such can be given. Especially regarding DNA-PK, the measurement of protein kinase activity of DNA-PK comprised in nuclear extracts by using the presence or absence of a double-stranded DNA fragment described above is expected to provide useful information for elucidating the relationship between DNA-PK and events related to the cell cycle or immunological functions.

It is known that the deficiency of DNA-PK in mice causes an immunodeficient mice called the SCID mice, and in a functional point of view, DNA-PK activity is closely related to immunity, the biological defense. The measurement of DNA-PK activity in the thymus and bone marrow, the place of differentiation of immunocompetent cells, is considered to be useful for estimating the probability of rejection following transplantation, or for estimating recovery following prognosis in patients given immuno-suppressants for any reason. Moreover, it is known that DNA-PK binds to a DNA end together with other proteins and is involved in cell cycle checkpoints and DNA repair. Therefore, there is a possibility that the measurement of DNA-PK activity together with ATM before radiation therapy to know the resistance of patients to radiation may become an important test item. The test sample at this time can be cells cultured from skin, or the like, of the patient or lesions of cancer, and such, and the activity before and after the irradiation is measured.

The present invention provides a method for measuring not only protein kinase activity, but also protein phosphatase activity towards a substrate peptide phosphorylated by a protein kinase selected from the group consisting of DNA-PK, ATM, and ATR. The method of this invention for measuring protein phosphatase activity comprises the following steps of,

(a) contacting a sample with a substrate peptide comprising at least the phosphorylation site of the phosphorylated protein and incubating the substrate peptide and the sample under conditions necessary for the dephosphorylation reaction, and,
(b) detecting a change in the phosphorylation level of the substrate peptide based on a change in reactivity of the substrate peptide with an antibody that identifies the phosphorylation state of the substrate peptide.

As the substrate peptide, the one used for the measurement of protein kinase activity can be phosphorylated beforehand and used. The phosphorylation of the substrate peptide is achieved by treating the substrate peptide with a protein kinase selected from the group consisting of DNA-PK, ATM and ATR, under conditions described above in which phosphorylation can be conducted. As for the substrate peptide necessary for the measurement of protein phosphatase activity, not only one synthesized enzymically, but also a chemically synthesized substrate peptide can be used as long it has an identical structure.

As for the antibody that can identify a phosphorylation state of the substrate peptide used in the measurement of the protein phosphatase activity, the same antibody as the one described above can be used. Moreover, the method for evaluating the phosphorylation level of the substrate peptide based on an immunoassay can be conducted based on a principle similar to the one described above. The only difference is that the relationship between the amplitude of the signal derived from the labeled peptide (or labeled antibody) in the measurement of protein phosphatase activity and the activity of the enzyme to be measured is inversed.

At present, no protein in the human living body has been identified as a protein that functions as a protein phosphatase towards a protein phosphorylated by a protein kinase selected from the group consisting of DNA-PK, ATM and ATR. However, the method of this invention for measuring protein phosphatase activity can be an assay system that measures the total protein phosphatase activity comprised in the tissue or cell extract, and such.

The method for measuring protein phosphatase activity of the present invention can treat various samples suspected to have the activity, similar to the method for measuring protein kinase activity. For instance, when attempting to purify a protein phosphatase contained in the extract, the protein phosphatase activity found in each fraction is useful as an index of enzyme purification.

Methods for measuring protein kinase activity or the protein phosphatase activity of the present invention can be used for screening inhibitors or promoters of protein kinase or protein phosphatase, respectively. Namely, the present invention relates to a method of screening for compounds that inhibit or promote the activity of a protein kinase or protein phosphatase. In the present invention, compounds that inhibit or promote these enzyme activities are generally designated as compounds that regulate enzyme activity.

The method of this invention of screening for a compound that inhibits or promotes the protein kinase activity of a protein kinase comprises the following steps of,

(a) contacting the protein kinase selected from the group consisting of DNA-PK, ATM, and ATR and a substrate peptide phosphorylated by the protein kinase and incubating the protein kinase and the substrate peptide under conditions necessary for the phosphorylation reaction in the presence of a test compound,
(b) detecting a change in phosphorylation level of the substrate peptide based on a change in reactivity of the substrate peptide with an antibody that identifies the phosphorylation state of the substrate peptide, and,
(c) selecting a compound that decreases or increases the change in the phosphorylation level of the substrate peptide by comparing with the phosphorylation level of the substrate peptide in the absence of the test compound.

The contact of the protein kinase and the substrate peptide, and the evaluation of the phosphorylation level of the substrate peptide can be conducted by a method similar to the one used in the measurement of protein kinase activity described above. As a result, a test compound used in the screening is judged to promote protein kinase activity when the detected amount of phosphate group(s) binding to serine residue (s) of the substrate peptide is decreased in the absence of the test compound.

As a protein kinase used in the screening of the present invention, not only DNA-PK, ATM or ATR of natural origin, but also enzyme proteins that can be obtained as recombinant proteins can be used. Moreover, not only an enzyme comprising an amino acid sequence similar to the natural enzyme, but also an enzyme fragment comprising only the protein kinase domain can be used. By using only the protein kinase domain, the expression product can be reduced, and as a result, a larger amount of enzyme protein can be easily obtained as a recombinant protein. For instance, it has been elucidated that protein kinase activity is expressed by only amino acids 2695-3056 (SEQ ID NO: 2) in the 3056 residues of full-length amino acid for human ATM, and amino acids 2305-2644 (SEQ ID NO: 3) in the 2644 residues of full-length amino acid for human ATR. In human DNA-PK, among the full length amino acids of 4127 residues, amino acid sequence from 3730-4127 shows a high homology with the amino acid sequence of the protein kinase domain of human ATM or ATR, and it is speculated that this region (SEQ ID NO: 1) corresponds to the protein kinase domain.

The method of the present invention for screening a compound that inhibits or promotes the phosphatase activity comprises the following steps of,

(a) contacting a protein phosphatase against the phosphorylated protein and a substrate peptide comprising at least a phosphorylation site of phosphorylated protein, and incubating the protein phosphatase and the substrate peptide under conditions necessary for the dephosphorylation reaction, in the presence of a test compound,
(b) detecting changes in dephosphorylation level of a substrate peptide based on a change in reactivity of the substrate peptide with an antibody that identifies the phosphorylation state of the substrate peptide, and,
(c) selecting a compound that decreases or increases changes in the dephosphorylation level of a substrate peptide by comparing with the dephosphorylation level of the substrate peptide in the absence of the test compound.

As a test compound used in these screening methods , for instance, peptides (including proteins), synthetic low molecular compounds, cellular extracts of animals, plants or bacteria, cell culture supernatants are used, but the test compound is not limited thereto. Moreover, for example, *E. coli* alkaline phosphatase, calf intestinal alkaline phosphatase, and protein phosphatases such as Cdc25 and Cdc14 can be used as the protein phosphatase.

The contact of protein phosphatase and the phosphorylated substrate peptide, and the evaluation of the phosphorylation level of the substrate peptide can be conducted by a method similar to the one used for measuring protein phosphatase activity described above. As a result, the test compound used in the screening is judged to inhibit the protein phosphatase activity when an increase in the amount of the phosphate group (s) binding to serine residue (s) of the substrate peptide is detected, compared with the detected amount of phosphate group(s) binding to serine residue(s) of the substrate peptide in the absence of the test compound (control). Contrastingly, the test compound used in the screening is judged to promote protein phosphatase activity when a decrease in the amount of the phosphate group (s) that bind to serine residue(s) of substrate peptide is detected.

In the screening method of the present invention, when cell extracts of animals, plants or bacteria, or cell culture supernatants are used as the test compound, a single compound which inhibits protein kinase (or protein phosphatase) activity can finally be specified by fractionating these test compounds by a method well known to one skilled in the art (for example, various chromatography) and detecting protein kinase or phosphatase activities of each of the fractions. Compounds isolated by these screenings, which inhibit or promote the protein kinase activity and protein phosphatase activity, are useful especially as candidate compounds for drugs for cancer therapy and antifungal antibiotics.

Additionally, the present invention relates to a kit comprising an antibody that identifies the phosphorylation state of a substrate peptide used in the assay or screening method described above. The kit of the present invention comprises, for example, a substrate peptide and buffer besides the above-mentioned antibody when using it for the detection of protein kinase activity. Moreover, the kit comprises a phosphorylated substrate peptide and buffer besides the above-mentioned antibody when using it for the detection of protein phosphatase activity. In addition, when using it for the screening of a compound that inhibits or promotes the activity of these enzymes, a protein kinase (or protein phosphatase) is further combined. Either of the substrate peptide or the above-mentioned antibody can be labeled as mentioned above, while the other can be solid-phased.

Standard enzyme and/or substrate peptide sample can be combined in the kits, for the calibration of the protein kinase (or protein phosphatase) activity and/or the assay system itself. Other components for stabilization, and such, can be added to these standard samples and above-mentioned antibody. For instance, 1% of BSA, and polyols such as sucrose and fructose with a final concentration of 0.2%~10% (desirably 1%) can be added to the standard sample as protein degeneration inhibitors after freeze-drying.

Brief Description of the Drawings

Figure 1 is a diagram showing the regulation of cell cycle by DNA damage checkpoints.
Figure 2 shows the reactivity of anti-phospho p53-S15.
Figure 3 shows the reactivity of anti-phospho p53-S33.
Figure 4 shows the reactivity of anti-phospho p53-S37.
Figure 5 shows the protein kinase activity of recombinant ATM detected by anti-phospho p53-S15 antibody.
Figure 6 shows the protein kinase activity of recombinant ATR detected by anti-phospho p53-S15 antibody.
Figure 7 shows the protein kinase activity of recombinant DNA-PK detected by anti-phospho p53-site specific antibody.

Best Mode for Carrying out the Invention

Herein below, the present invention is explained more in detail based on examples.

[Example 1] Preparation of anti-phosphorylated peptide antibody

1) Preparation of immunogen

1. Preparation of peptide

The following six peptides comprising Ser15, Ser33 and Ser37 of human p53, respectively, which are reported to be phosphorylation sites, were made using a peptide synthesizer.

Phospho p53-S15: SVEPPLS (p) QETFSDC (SEQ ID NO: 4)
p53-S15 : SVEPPLSQETFSDC (SEQ ID NO: 5)
Phospho p53-S33: PENNVLS (p) PLPSQAC (SEQ ID NO: 6)
p53-S33 : PENNVLSPLPSQAC (SEQ ID NO: 7)
Phospho p53-S37: VLSPLPS (p) QAMDDLC (SEQ ID NO: 8)
p53-S37 : VLSPLPSQAMDDLC (SEQ ID NO: 9)

The above-mentioned peptide sequence is described in one-letter expression from the amino terminus to the direction of the carboxy terminus. S (p) indicates a phosphorylated serine residue. The purity was confirmed to be of 90% or more by HPLC. Phospho p53-S15 and p53-S15 correspond to the amino acid sequence from amino acid residues 9-21 of human p53. Phospho p53-S33 and p53-S33 correspond to amino acid sequence from amino acid residues 27-39 of human p53 and Phospho p53-S37 and p53-S37 correspond to amino acid sequence from amino acid residues 31-43 of human p53. All cysteine residues at the carboxy terminus of peptides were introduced for the covalent binding of the peptides to carrier proteins.

2. Binding of peptides to career proteins

Phosphorylated peptides (phospho p53-S15, -S33, or -S37) were covalently bound separately to keyhole limpet hemocyanin (KLH) to make immunogens. m-maleimidobenzoyl-N-hydroxysuccinimide ester (MBS) was used as a cross-linker for the binding of these peptides and KLH. Equivalent amounts of KLH and peptide were crosslinked. This peptide-KLH conjugate was used as the immunogen.

3. Preparation of immunogen and method of immunization

20 µg (100 µl) of carrier protein KLH and peptide conjugate (peptide-KLH) was used as the immunogen for rabbits, and 10 µg (100 µl) was used as the immunogen for mice as the immunogen for a single immunization. 100 µl of Freund's complete adjuvant and peptide-KLH (100 µl each) were mixed in a 1.5ml tube until the mixture was completely emulsified using a 1 ml syringe and a 21-gauge needle.

Phospho p53-S15-KLH or the emulsion of phospho p53-S15-KLH and adjuvant was injected intraperitoneally to mice (Balb/c) using a 26-gauge needle (immunization). The immunization was carried out four times in total once every week. At the fourth immunization, 50~100 µl of blood was collected from the venous plexus at the eye pit and the antibody titer was checked by ELISA.

Furthermore, the emulsion of phospho p53-S37-KLH and adjuvant was immunized subcutaneously to 7-8 sites at the back of a rabbit (Japanese white) using a 26-gauge needle. The immunization was carried out five times in total once every week. At the fifth immunization, several milliliters of blood were collected from the ear lobule vein of the rabbit, and the antibody was checked by ELISA.

2) Preparation of anti-phospho p53-S15 monoclonal antibody and anti-phospho p53-S33 monoclonal antibody

1. Preparation of hybridoma

After confirming a sufficient antibody titer, spleen was removed from the mice and spleen cells were fused with myeloma cells using polyethylene glycol. The selection of hybridomas was carried out by HAT (hypoxanthine, aminopterin, thymidine) selection. Clones having a higher reactivity towards a phosphorylated peptide (phospho p53-S15, phospho p53-S33) than towards an unphosphorylated peptide (p53-S15, p53-S33) were screened by ELISA using the culture supernatants of hybridomas.

2. Obtaining ascites and purifying the antibody

Hybridoma clones obtained by limiting dilution was cultured in 75 cm$^2$ flasks, and were injected intraperitoneally to mice. 1 ml and 0.5 ml of pristane (Sigma) was injected to mice 10 days and 3 days before injecting hybridoma, respectively. One or two weeks later, the ascites was collected while observing the condition of the mice. Ammonium sulfate was added to the ascites to a final concentration of 50%, and stirred for more than 1 hour at 4°C. The precipitate was collected by rapid centrifugation. After completely dissolving the precipitate with a minimum amount of pure water, it was dialyzed against PBS using a dialysis membrane. After completely equilibrating to PBS, complexes with protein A sepharose (Pharmacia) were formed. Finally, the antibody was eluted from protein A sepharose.

3) Preparation of anti-phospho p53-S37 polyclonal antibody

1. Collection of whole blood from rabbits

After confirming a sufficient antibody titer, a 3-week cycle of collecting blood, rest, and immunization, was carried out and was repeated four times, starting from the week after confirming the antibody titer. Blood was collected from the ear lobule vein, similar to the confirmation of the antibody titer. Approximately 60-70 ml of blood was collected per time. During blood collection at the fifth cycle, as much blood as possible was collected using a catheter.

2. Recovery and storage of rabbit serum and separation of antibody fraction

The collected blood was kept overnight at 4°C to make them clot, and the serum was separated. Sodium azide was added to the serum to a final concentration of 0.1% and stored at 4°C.

To separate and concentrate the antibody fraction, ammonium sulfate was added to the recovered serum to a final concentration of 50%, and stirred at 4°C more than 1 hour. The precipitation was collected by rapid centrifugation. After completely dissolving the precipitation with a minimum amount of pure water, it was dialyzed against PBS using a dialysis membrane. After completely equilibrating to PBS, the antibody fraction was subjected to column chromatography and the antibody was purified.

3. Preparation of specified column and absorption column

Peptide was bound to Sepharose 4B by mixing 1~2 g CNBr-activated Sepharose 4B and 1 mg peptide in 5~10 ml of 0.1 M carbonate buffer at 4°C overnight using a rotator. The next day, the column was filled with Sepharose 4B and washed with PBS of 4~10 the volume of the column. After washing, the column was equilibrated with 1 M Tris-HCl (pH 7.0), and left to stand more than 1 hour at 30°C to block active groups remaining on the surface of Sepharose 4B. After blocking, the column was washed with PBS, equilibrated, and then used. Phospho p53-S37 was used for making the specified column and p53-S37 was used for making the absorption column, respectively.

4. Purification of anti-phospho p53-S37 antibody using the specification column and absorption column

To purify the anti-phosphorylated peptide antibody, the anti-phospho p53-S37 antibody fraction was passed through the specification column. After washing with PBS-0.1% Tween 20, anti-phosphorylated peptide antibody bound to the column was eluted by 0.1 M glycine-HCl (pH 3.0).

Anti-phospho p53-S37 fraction eluted from the specified column was passed through the absorption column and p53-S37 Sepharose 4B column. By passing through the absorption column, antibodies in the antibody fraction that react also to unphosphorylated peptide is absorbed to the column. Anti-phosphorylated peptide-specific antibody does not bind to the column and passes through. The absorption column was washed with PBS-0.1% Tween 20, and the anti-unphosphorylated peptide antibody bound to the column was eluted with 0.1 M glycine-HCl (pH 3.0). After the elution, the column was re-equilibrated with PBS-0.1% Tween 20. The antibody fraction was repeatedly passed through the absorption column until the anti-unphosphorylated peptide antibody was completely absorbed. The progress of absorption was monitored by ELISA using an unphosphorylated peptide-sensitized plate.

After dialyzing the antibody absorbed by the absorption column (anti-phosphorylated peptide specific antibody) against PBS, the specificity of the antibody towards a phosphorylated peptide was finally confirmed by ELISA using a phosphorylated peptide-sensitized plate.

4) Testing of antibody

1. Preparation and the use of ELISA plate for testing the titer and specificity of the antibody

Peptide was dissolved in PBS to a concentration of 10 µg/ml, and 50 µl/well were seeded into a microtiter plate for ELISA, and sensitized overnight at 4°C. After sensitization, peptide solution was removed and 200 µl/well of 1% BSA-0.1% Tween 20-PBS was added, and blocking was done for more than 1 hour at room temperature.

Thus prepared phosphorylated peptide (phospho p53-S15, phospho p53-S33)-sensitized plates were used for the measurement of antibody titer, absorption of anti-phosphorylated serine residue-specific antibody, and the confirmation of specificity of anti-phosphorylated peptide antibody. Moreover, unphosphorylated peptide (p53-S15, p53-S33, or p53-S37) sensitized plates were used for verifying the absorption of non-specific antibody by the column.

Serum and antibodies were diluted with 0.1% Tween 20-PBS if necessary. The diluted samples were seeded onto the sensitized plate at 100 µl/well and left to stand for 1 hour (primary reaction) . After the primary reaction, each well was washed thoroughly for more than 4 times with PBS using washing bottle. 3000-fold diluted horseradish peroxidase (HRP)-labeled goat anti-rabbit IgG (H+L) antibody (MBL, 458) with 0.1% Tween 20-PBS was seeded at 100 µl/well, and the plate was left to stand for 1 hour at room temperature (secondary reaction). After the secondary reaction, the wells were washed similarly with PBS and then 0.8 mM TMB (Tetramethylbenzidine) solution was added at 100 µl/well, and the color was developed for 5-20 min at 30°C (color reaction). The color reaction was stopped by adding 100 µl/well of 1.5 N $H_3PO_4$ and the absorbance at 450 nm was measured using a microtiter plate reader.

2. Confirmation of specificity

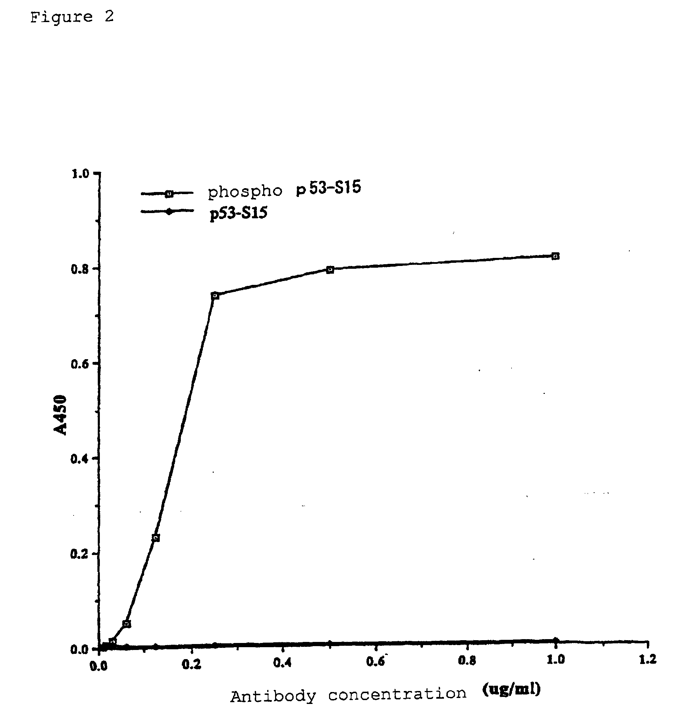

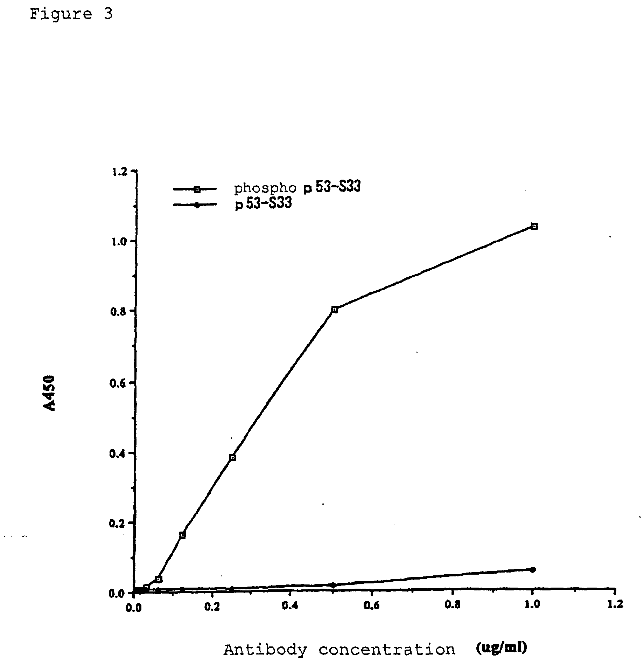

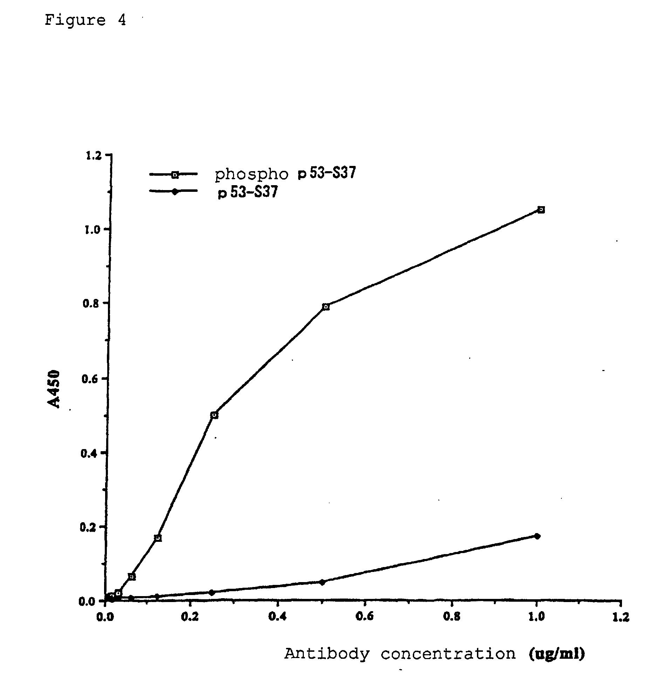

Anti-phospho p53-S15-specific antibody, anti-phospho p53-S33-specific antibody and anti-phospho p53-S37-specific antibody were diluted and their specificities were confirmed using plates sensitized with the corresponding peptide (Figures 2-4).

[Example 2] Preparation of recombinant ATM and ATR

1) cDNA cloning of ATM and ATR by PCR method

1. Designing of primers for amplification of ATM and ATR

To amplify the cDNA sequence encoding the protein kinase domain (2695-3056, SEQ ID NO: 2) of human ATM, hATM-F1 and hATM-R1 primers described below were designed. Similarly, hATR-F1 and hATR-R1 primers described below were designed for the amplification of cDNA sequence encoding the protein kinase domain of human ATR (2305-2644, SEQ ID NO: 3).

<Primers for ATM amplification>

Forward primer (hATM-F1); 5 '- GCG AAT TCA GGT GTA AAT TTA CCA AAA ATA-3' (SEQ ID NO: 10) (Two bases (GC) at 5' side for easy digestion with restriction enzyme. Bases 3-8 (GAATTC) at 5' side for EcoRI restriction site.)
Reverse primer (hATM-R1); 5 '- GCG CTC GAG CAC CCA AGC TTT CCA TCC TGG-3' (SEQ ID NO: 11) (Three bases (GCG) at 5' side for easy digestion with restriction enzyme. Bases 4-9 (CTCGAG) at 5' side for XhoI restriction site.)

<Primers for ATR amplification>

Forward primer (hATR-F1); 5 '- GCG GGA TCC TCT CTT CAG AAA CCA AAG AAG-3' (SEQ ID NO: 12) (Three bases (GCG) at 5' side for easy digestion with restriction enzyme. Bases 4-9 (GGATCC) at 5' side for BamHI restriction site.)
Reverse primer (hATR-R1); 5 '- GCG CTC GAG CAT ATA TGG AGT CCA ACC AAG-3' (SEQ ID NO: 13) (Three bases (GCG) at 5' side for easy digestion with restriction enzyme. Bases 4-9 (CTCGAG) at 5' side for XhoI restriction site.)

2. Preparation of template DNA

As the template for the amplification of protein kinase domain of ATM and ATR genes by PCR method, cDNA of human breast cancer-derived ZR75-1 cells was used. First, total RNA was extracted and purified by using phenol-thiocyanic acid guanidine method (Nippon Gene, Isogen) to construct cDNA. cDNA was synthesized from the extracted total RNA, using oligo-dT or oligo-random primers (reverse transcription).

3. Condition of PCR

The PCR was carried out under the following conditions having three stages.

1; 1 cycle of 95°C 5 min (denaturing), 52°C 1 min (annealing), and 72°C 2 min (extension),
2; 35 cycles of 94°C 30 sec (denaturing), 62°C 30 sec (annealing), and 72°C 2 min (extension),
3; 1 cycle of 72°C, 10 min,

4. Cloning to expression vector.

PCR product amplified by hATM-F1 and hATM-R1 primer set was digested with EcoRI and XhoI, and PCR product amplified by hATR-F1 and hATR-R1 primer set was digested with BamHI and XhoI, respectively. Restriction enzyme-digested DNA was subjected to agarose gel electrophoresis and purified with Geneclean (BIO101). Purified DNA was ligated into the expression vector pcDNA3-6myc, which had been digested beforehand with BamHI or EcoRI, and XhoI, same restriction enzymes as those used to digest the ends of PCR products. Since pcDNA3-6myc expression vector adds 6x myc-tag to the N-terminal side, the protein is expressed extracellularly as 6myc-ATM or 6myc-ATR proteins. After ligation, *E. coli* (DH5$\alpha$) was transformed according to the methods described in "Molecular Cloning (Sambrook et al. the second edition (1989) Cold Spring Harbor Laboratory Press, Cold Spring Harbor, NY)", and several single colonies were obtained.

5. Confirmation of nucleotide sequences

According to the methods described in "Molecular Cloning (described above)", plasmid DNA was purified from each single colony. The nucleotide sequences of the insert DNA were determined by an auto-sequencer based on the Sanger's method. By comparing the sequence of insert DNA and that published in the database, the cDNA sequences of ATM and ATR were confirmed.

2) Measuring of enzyme activities of ATM and ATR proteins encoded by the cDNA clones

1. Preparation of substrate for activity measurement

ATM and ATR are known to phosphorylate the serine residue, Ser15, of human p53. Therefore, cDNA sequence encoding 1-99 amino acids of human p53 was inserted to pGEX expression vector, and *E. coli* was transformed with the vector. A fusion protein GST-p53 (1-99), fused with glutathione s-transferase (GST) expressed by this *E. coli*, was used as the substrate for measuring activities of ATM and ATR.

In the case of pGEX vector, a recombinant protein is produced as a fusion protein with GST. The recombinant protein was purified by taking advantage of the fact that this GST enzyme has an affinity for glutathione (GSH). After confirming the expression of the recombinant protein, the cells were suspended in % Tween 20-PBS and then disrupted by ultrasonication. After rapid centrifugation, the supernatant was taken as the soluble fraction containing the recombinant protein. The soluble fraction was passed through a GSH-Sepharose 4B column (Pharmacia) and GST-p53 (1-99) the protein was absorbed to the column. The column was washed with WE buffer (10 mM 2-mercaptoethanol, 2 mM $MgCl_2$, 20 mM Tris-HCl pH 7.5) and then the recombinant protein was eluted with G buffer (10 mM GSH, 50 mM Tris-HCl pH 9.6). The GST-p53 protein eluted was dialyzed against PBS.

2. Transfection to human 293T cells and activity measurement

Plasmid DNA comprising ATM or ATR as insert was transfected to human 293T cells using Trans IT-LT1 (Mirus). After 2 days, cells washed with ice-cold PBS were suspended in HGN buffer (10 mM HEPES (pH 7.5), 10% glycerin, 150 mM NaCl, 1% Tween 20, 10 mM β-glycerophosphoric acid, 1 mM NaF, 0.1 mM $NaVO_4$, 2 μg/ml pepstatin A, 5 μg/ml leupeptin, 10 μg/ml aprotinin, 2 mM benzamidine, 1 mM PMSF, 1 mM DTT). Supernatant following the centrifugal separation was designated as the cell extract.

The cell extract was subjected to SDS-polyacrylamide gel electrophoresis (PAGE) and then western blotting analysis was carried out. The expression of the protein was confirmed using anti-myc tag antibody (MBL).

Anti-myc tag antibody and protein A Sepharose (Pharmacia) were added to the cell extract for the immunoprecipitation of 6myc-ATM protein or 6myc-ATR protein. Immunoprecipitates were washed with 100 mM Tris (pH 7.5), 0.5 M LiCl, and then further washed with 1x ATM/ATR reaction buffer (10 mM HEPES (pH7.5), 50 mM β-glycerophosphoric acid, 50 mM NaCl, 10 mM MgCl2, 10 mM $MnCl_2$, 5 mM ATP, 1 mM DTT). Washed Immunoprecipitates were incubated in phosphorylation reaction buffer with 370 kBq γ-$^{32}$P-ATP, 1 μg GST-p53 (1-99), for 30 min at 30°C. The reaction solution was subjected to SDS-PAGE, and by taking an autoradiogram of the dried gel, the incorporation of $^{32}$P into the GST-p53 (1-99) used as substrate was confirmed.

3) Purification of recombinant protein from Spodoptera frugiperda (Sf9) cells

1. Preparation of recombinant baculovirus solution

For cDNA clones in which GST-p53 (1-99) is labeled with $^{32}$P, the inserts were subcloned into pFASTBACHT vector (GIBCO BRL) after cutting the inserts out using EcoRI and XhoI for ATM, BamHI and XhoI for ATR. Plasmid clones of pFASTBACHT were transformed to *E. coli* (DH10BAC strain) and they were grown on Luria agar plates (10 g/L peptone, 5 g/L yeast extract, 10 g/L NaCl, 12 g/L agar, 200 μg/ml X-Gal, 40 μg/ml IPTG, 10 μg/ml tetracycline, 7 μg/ml gentamicin, 50 μg/ml kanamycin). In DH10BAC, the expression cassette within pFASTBACHT transposes to bacmid DNA by site-specific transposition. The recombinant bacmid DNA was purified from white colonies in which transposition occurred. Sf9 cells were transformed with recombinant bacmid DNA using CELLFECTIN (GIBCO BRL).

Since recombinant baculoviruses are produced into the culture medium, the culture medium was separated by centrifugation after 1 week and the supernatant was taken as the virus solution. Basically, the operation described above was conducted according to the BAC-TO-BAC Baculovirus Expression System (GIBCO BRL).

2. Purification of recombinant protein

Sf9 cells were cultured to an almost confluent state in 75 $cm^2$ flasks, and recombinant baculovirus solution was added to the culture medium. Three or four days after infection, cells were suspended in ice-cold PBS and washed by centrifugation. Cells were suspended-in HGN buffer (-DTT) and supersonicated on ice. Next, cells were centrifuged and the resultant supernatant was used for purification as the soluble fraction. In the expression cassette of pFASTBACHT vector, 6 His residues (6x His) are added in the N terminal side. After western blotting analysis was carried out using a portion of the extract, the expression of the protein was confirmed using anti-5x His antibody (GIAGEN). The soluble fraction was passed through Ni-chelating Sepharose column (Pharmacia), and the recombinant protein was absorbed to the column by its 6x His-Tag. Since 6x His-Tag forms a complex with $Ni^{2+}$, recombinant protein is absorbed to $Ni^{2+}$ on the column. After washing the column with HGN buffer (-DTT), the recombinant protein was eluted by elution buffer (50 mM~1 M imidazole, 0.5 M NaCl, 20 mM Tris-HCl pH 7.9). In that case, the concentrations of imidazole were changed stepwise to 10 mM, 25 mM, 50 mM, 100 mM, 250 mM, 500 mM, 1 M, for the elution.

For each imidazole fraction, the fraction comprising recombinant protein as detected by western blotting with anti-5x His antibody was dialyzed against a dialysis buffer (50 mM HEPES (pH 7.5), 1 mM DTT, 1 mM EDTA (pH 8.0), and 50% glycerin) overnight.

Samples dialyzed were then passed through a MonoQ column (Pharmacia). Using FPLC (Pharmacia), the absorbed protein was eluted with a concentration gradient using 0~1 M NaCl-containing 10 mM Tris-HCl (pH 7.5), 1 mM DTT, and 10% glycerin. The fraction with the recombinant protein as detected by western blotting using the anti-5x His antibody, was dialyzed against a dialysis buffer.

3. Purity test of purified recombinant protein as a protein kinase

The protein kinase activity of the dialyzed fraction containing recombinant protein was measured. Using 2~5 ml of sample concentrated by dialysis, 1x ATM/ATR reaction buffer containing 0.5 mM peptide, 0.1 mM ATP (containing 185 kBq $\gamma$-$^{32}$P-ATP), was prepared and incubated for 10 min at 30°C. Next, the reaction solution was spotted onto a P81 phosphocellulose filter paper (Whatman) and was washed with 75 mM $H_3PO_4$. After three 20 min washings, the filter paper was dried and the radioactivity of $^{32}$P incorporated into the peptide was measured using a scintillation counter (Beckman) by Chelenkov's method.

DNA-PK peptide (Promega) was used for measuring the activity of ATM and ATR, Kemptide (Promega) was used for measuring the activity of PKA, and Casein Protein kinase II Peptide (NEB) was used for measuring the activity measurement of casein protein kinase II. As a result, purified recombinant ATM and ATR did not show PKA or casein protein kinase II.

4. Confirmation of phosphorylation site specificity of recombinant ATM and ATR

1x ATM/ATR reaction buffer containing recombinant ATM or ATR, and GST-p53 (1-99) described above was incubated at 30°C, and periodically, a portion of the buffer was mixed with an equivalent volume of 2x SDS buffer to stop the reaction. These periodical reaction products were subjected to SDS-PAGE and western blotting. The primary antibody (anti-phospho p53-S15 monoclonal antibody, anti-phospho p53-S33 monoclonal antibody or phospho p53-S37 monoclonal antibody) was diluted to 0.5 $\mu$g/ml with an antibody dilution buffer (1% BSA, 0.1% $NaN_3$) before use. After the primary antibody reaction, the membrane was washed with 0.05% Triton X100-PBS. 3000-fold diluted HRP-labeled goat anti-mouse antibody (MBL) or 2000-fold diluted HRP-labeled goat anti-rabbit antibody (MBL) diluted with 0.05% Triton X100-PBS was subjected to the antigen-antibody reaction (secondary reaction). After washing the membrane with 0.05% Triton X100-PBS, the detection of the phosphorylation site was conducted using ECL detection kit (Amersham). As a result, it was confirmed that recombinant ATM and ATR phosphorylate the S15 residue of GST-p53 (1-99), but do not phosphorylate S33 and S37 residues. Taken together, it was confirmed that recombinant ATM and ATR phosphorylate SQE sequence site-specifically.

[Example 3] System for measuring activity of DNA-PK, ATM, and ATR

1) Construction of an ELISA procedure for measuring activity of ATM or ATR

1. Preparation of GST-p53 (1-99)-sensitized plate

GST-p53 (1-99) was dissolved in PBS to a concentration of 20 $\mu$g/ml, and seeded onto a microtiter plate for use in ELISA at 50 $\mu$l/well and sensitized overnight at 4°C. After sensitization, GST-p53 (1-99) solution was removed and 200 $\mu$l/well of 1% BSA-0.1% Tween 20-PBS was added and blocking was done for more than 1 hour at room temperature.

2. Procedure for measuring activity of recombinant ATM or ATR

After a protein phosphorylation reaction was carried out in the wells in which recombinant protein GST-p53 (1-99) has been solid-phased, ELISA was carried out serially in the same wells.

1x ATM/ATR reaction buffer containing recombinant ATM or ATR was prepared and added onto GST-p53 (1-99)-sensitized plates at 50 $\mu$l/well (protein phosphorylation reaction). After incubating for a certain time period at 30°C, 200 $\mu$l of 75 mM $H_3PO_4$ solution was added to each well to stop the reaction. Each well was washed sufficiently with PBS more than 4 times . The primary antibody (anti-phospho p53-S15 monoclonal antibody) was diluted with an antibody dilution buffer (1% BSA, 0.1% NaN$_3$) to the concentration of 0.5 µg/ml, and added to each well after washing at 100 µl/well and the plate is left to stand for 1 hour at room temperature (primary reaction) . After the primary reaction, wells were washed similarly with PBS. 100 µl/well of 3000-fold diluted HRP-labeled goat anti-mouse antibody (MBL) diluted with 0.05% Triton X100-PBS was added to each well and further left standing for 1 hour at room temperature (secondary reaction) . After washing with PBS, 100 µl of HRP substrate was added to each well and the color was developed at 37°C. The color reaction was stopped by adding 100 µl/well of 1.5 N H$_3$PO$_4$, and then, absorbance was measured at 450 nm using a microtiter plate reader.

3. Assay results

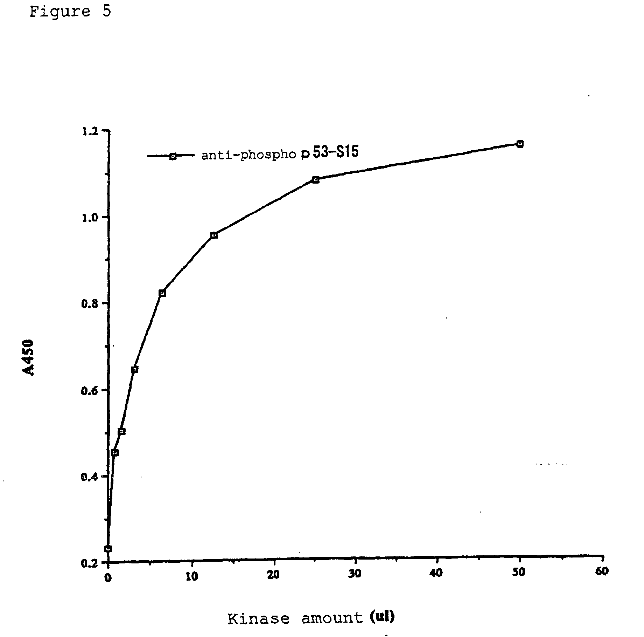

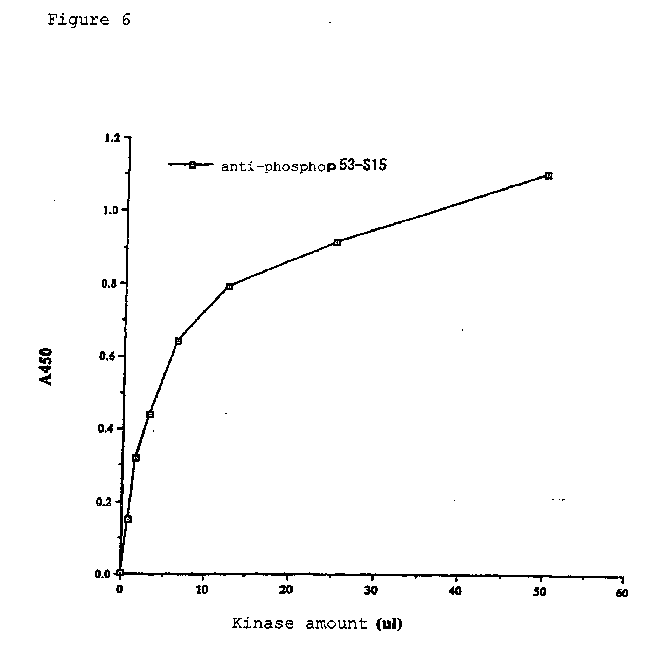

Results of measuring protein kinase activity of recombinant ATM and ATR using the ELISA method described above are shown (Figures 5 and 6). Both ATM (Figure 5) and ATR (Figure 6) showed an increase of absorbance depending on the concentration of the enzymes. It was shown that activity of these enzymes could be measured based on the present invention.

2) Construction of an ELISA for measuring DNA-PK activity

1. Confirmation of phosphorylation site specificity of DNA-PK

1x DNA-PK reaction buffer (25 mM HEPES pH 7.5, 75 mM KCl, 10 mM MgCl$_2$, 1 mM DTT, 0.2 mM EGTA, 0.25 mM ATP, 10 µg/ml ultrasonicated double-strand DNA, 50 mM β-glycerophosphoric acid) containing DNA-PK (Promega) and GST-p53 (1-99) was incubated at 30°C, and periodically, a portion of the reaction solution was mixed with an equivalent volume of 2x SDS buffer to stop the reaction. These periodical reaction samples were subjected to SDS-PAGE and western blotting. The primary antibody (anti-phospho p53-S15 monoclonal antibody, anti-phospho p53-S33 monoclonal antibody or phospho p53-S37 monoclonal antibody) was diluted to a concentration of 0.5 µg/ml with an antibody dilution buffer (PBS, 1% BSA, 0.1% NaN$_3$) before use. After the primary antibody reaction, the membrane was washed with 0.05% Triton X100-PBS. 3000-fold diluted HRP-labeled goat anti-mouse antibody (MBL) or 2000-fold diluted HRP-labeled goat anti-rabbit antibody (MBL) diluted with 0.05% Triton X100-PBS was subjected to the antigen-antibody reaction (secondary reaction) . After washing the membrane with 0.05% Triton X100-PBS, the phosphorylation sites were detected using the ECL detection kit (Amersham) . As a result, it was confirmed that DNA-PK phosphorylates the S15 and S37 residues of GST-p53 (1-99) but does not phosphorylate the S33 residue. Taken together, it was confirmed that DNA-PK phosphorylates the SQ sequence site specifically.

2. Preparation of GST-p53 (1-99)-sensitized plate

GST-p53 (1-99) was dissolved in PBS to a concentration of 20 µg/ml, and seeded into a microtiter plate for ELISA at 50 µl/well and sensitized overnight at 4°C. After sensitization, GST-p53 (1-99) solution was removed and 200 µl/well of 1% BSA-0.1% Tween 20-PBS was seeded into the wells and blocking was done for more than 1 hour at room temperature.

3. Procedure for measuring DNA-PK activity

After the protein phosphorylation reaction was carried out in the wells in which recombinant protein GST-p53 (1-99) has been solid phased, ELISA was carried out in the same wells.
DNA-PK reaction buffer containing DNA-PK was prepared and added into GST-p53 (1-99)-sensitized plates at 50 µl/well (protein phosphorylation reaction) . After incubating for a certain time period at 30°C, 200 µl of 75 mM H$_3$PO$_4$ solution was added to each well to stop the reaction. Each well was washed sufficiently with PBS more than 4 times. The primary antibody (anti-phospho p53-S15 monoclonal antibody or anti-phospho p53-S37 polyclonal antibody) was diluted with an antibody dilution buffer (1%BSA, 0.1%NaN3) to the concentration of 0.5 µg/ml, and added to each well after washing at 100 µl/well and left to stand for 1 hour at room temperature (primary reaction) . After the primary reaction, wells were washed similarly with PBS. 100 µl/well of HRP-labeled goat anti-mouse antibody (MBL) diluted 3000-fold or HRP-labeled goat anti-rabbit antibody (MBL) diluted 2000-fold with 0.05% Triton X100-PBS was added to each well and further left to stand for 1 hour at room temperature (secondary reaction). After washing with PBS, 100 µl of HRP substrate solution was added to each well and the color was developed at 37°C. The color reaction was stopped by adding 100 µl/well if 1.5 N H$_3$PO$_4$, and then, absorbance at 450 nm was measured using a microtiter plate reader.

4. Assay result

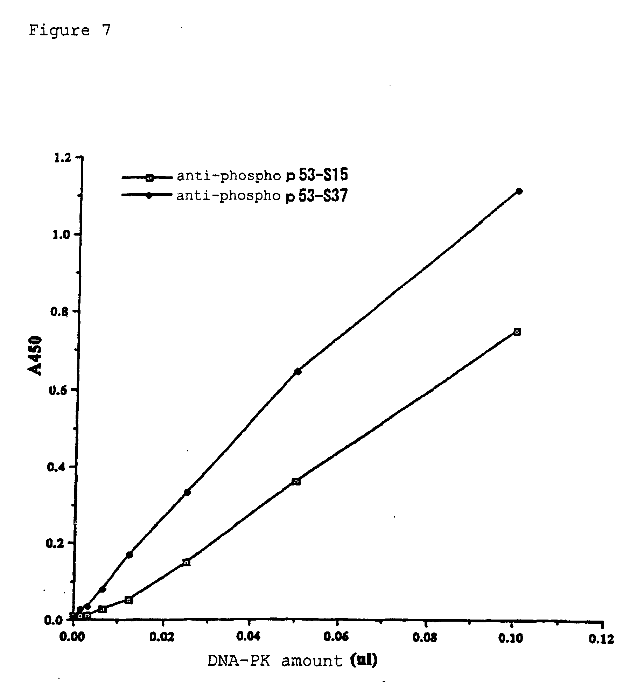

The protein kinase activity of DNA-PK was measured using the ELISA method described above (Figure 7). DNA-PK showed an increase in absorbance depending on the concentration of the enzyme. It was shown that activity of DNA-PK could be measured based on the present invention.

Industrial Applicability

As shown in Examples, protein kinase activity of DNA-PK, ATM, and ATR could be measured by ELISA using an anti-phosphorylated peptide specific antibody. Up to now, protein kinase activity was measured using $\gamma$-$^{32}$P-ATP labeled with the radioisotope $^{32}$P, and therefore, procedures for treating waste fluid, and special facilities, and such, were necessary. However, since the activity assay system shown in the Examples does not use radioisotopes, simple, easy, and rapid search of inhibitors can be conducted in ordinary laboratories by adding various chemically synthesized materials, extracts of cells and bacteria, culture supernatants, and such, to the enzyme reaction in this assay system.

Moreover, though DNA-PK, ATM, and ATR are serine/threonine protein kinases belonging to PI3K family with similar primary structures, their activities can be distinguished by analyzing the phosphorylation of Ser15 and Ser37 in p53, as shown in examples. The present examples can provide an opportunity to generate molecular probes of each enzyme.

**Claims**

1.  A method for measuring protein kinase activity of a protein kinase in a sample, wherein the protein kinase is selected from the group consisting of DNA-PK, ATM, and ATR, and said method comprises the following steps of,

    (a) contacting the sample with a substrate peptide phosphorylated by the protein kinase under conditions necessary for the phosphorylation reaction, and,
    (b) detecting a change in the phosphorylation level of the substrate peptide based on a change in reactivity of the substrate peptide with an antibody that identifies the phosphorylation state of the substrate peptide.

2.  A method of screening for a compound that inhibits or promotes the protein kinase activity of a protein kinase, wherein the protein kinase is selected from the group consisting of DNA-PK, ATM, and ATR, and wherein the method comprises the following steps of,

    (a) contacting the protein kinase selected from the group consisting of DNA-PK, ATM, and ATR and a substrate peptide phosphorylated by the protein kinase and incubating the protein kinase and the substrate peptide under conditions necessary for the phosphorylation reaction in the presence of a test compound,
    (b) detecting a change in phosphorylation level of the substrate peptide based on a change in reactivity of the substrate peptide with an antibody that identifies the phosphorylation state of the substrate peptide, and,
    (c) selecting a compound that decreases or increases the change in the phosphorylation level of the substrate peptide by comparing with the phosphorylation level of the substrate peptide in the absence of the test compound.

3.  A method for measuring protein phosphatase activity of a protein phosphatase against a phosphorylated protein in a sample, wherein the protein is phosphorylated by a protein kinase selected from the group consisting of DNA-PK, ATM, and ATR, and wherein the method comprises the following steps of,

    (a) contacting a sample with a substrate peptide comprising at least the phosphorylation site of the phosphorylated protein and incubating the substrate peptide and the sample under conditions necessary for the dephosphorylation reaction, and,
    (b) detecting a change in the phosphorylation level of the substrate peptide based on a change in reactivity of the substrate peptide with an antibody that identifies the phosphorylation state of the substrate peptide.

4.  A method of screening for a compound that inhibits or promotes the protein phosphatase activity of a protein phosphatase against a phosphorylated protein, wherein the protein is phosphorylated by a protein kinase selected from the group consisting of DNA-PK, ATM, and ATR, and wherein the method comprises the following steps of,

    (a) contacting a protein phosphatase against the phosphorylated protein and a substrate peptide comprising at least a phosphorylation site of phosphorylated protein, and incubating the protein phosphatase and the substrate peptide under conditions necessary for the dephosphorylation reaction, in the presence of a test compound,
    (b) detecting changes in dephosphorylation level of a substrate peptide based on a change in reactivity of the substrate peptide with an antibody that identifies the phosphorylation state of the substrate peptide, and,
(c) selecting a compound that decreases or increases changes in the dephosphorylation level of a substrate peptide by comparing with the dephosphorylation level of the substrate peptide in the absence of the test compound.

5. A method according to any one of claim 1 to claim 4, wherein the substrate peptide comprises an amino acid sequence comprising the SQ motif or the SQE motif.

6. The method according to claim 1, wherein the method separately measures protein kinase activities of several protein kinases coexisting in a sample, wherein each protein kinase phosphorylates a different site of the substrate peptide, and wherein the antibody that identifies the phosphorylation state identifies a phosphorylation site.

7. The method according to claim 6, wherein the protein kinase is ATM or ATR, and the antibody is an antibody that identifies the phosphorylation state of the SQE motif comprising 15th serine of p53.

8. The method according to claim 6, wherein the protein kinase is DNA-PK, and the antibody is an antibody that identifies the phosphorylation state of the SQ motif comprising serine 37 of p53.

9. A method for measuring DNA-PK activity, wherein the method comprises the following steps of,

(a) measuring the protein kinase activity in a sample by the method according to claim 1 in the absence of double-stranded DNA,
(b) measuring the protein kinase activity in a sample by the method according to claim 1 in the presence of double-stranded DNA, and,
(c) determining the protein kinase activity derived from DNA-PK by comparing results of (a) and (b).

10. A method according to any one of claim 1 to claim 4, wherein the reactivity of the antibody towards a phosphorylated substrate peptide is higher than the reactivity towards an unphosphorylated substrate peptide.

11. A method according to any one of claim 1 to claim 4, wherein the reactivity of the antibody towards an unphosphorylated substrate peptide is higher than the reactivity towards a phosphorylated substrate peptide.

12. A method according to any one of claim 1 to claim 4, wherein the substrate peptide is labeled.

13. A method according to any one of claim 1 to claim 4, wherein the substrate is solid-phased.

14. A method according to any one of claim 1 to claim 4, wherein the antibody is labeled.

15. A method according to any one of claim 1 to claim 4, wherein the phosphorylation level of the substrate peptide is evaluated by ELISA.

16. A compound that regulates the activity of a protein kinase selected from the group consisting of DNA-PK, ATM, and ATR, wherein the compound is isolated by the method of screening according to claim 2.

17. A compound that regulates the protein phosphatase activity of a protein phosphatase towards a protein phosphorylated by a protein kinase selected from the group consisting of DNA-PK, ATM, and ATR, wherein the compound is isolated by the method of screening according to claim 4.

18. A compound according to claim 16 or claim 17, wherein the compound is natural.

19. An antibody for use in a method according to any one of claim 1 to claim 4, wherein the method identifies the phosphorylation state of a substrate peptide.

20. A kit for a method according to any one of claim 1 to claim 4, comprising an antibody that identifies the phosphorylation state of a substrate peptide.

Figure 1

Figure 2

Figure 3

Figure 4

Figure 5

Kinase amount (ul)

Figure 6

Figure 7

<table>
<tr><td colspan="2" align="center"><b>INTERNATIONAL SEARCH REPORT</b></td><td colspan="2">International application No.<br>PCT/JP00/03193</td></tr>
</table>

| | |
|---|---|
| A. CLASSIFICATION OF SUBJECT MATTER<br>Int.Cl$^7$ G01N33/53, G01N33/573, C07K16/40, C12Q1/48<br>C12N15/54, C12N9/12 | |

According to International Patent Classification (IPC) or to both national classification and IPC

| |
|---|
| B. FIELDS SEARCHED |
| Minimum documentation searched (classification system followed by classification symbols)<br>Int.Cl$^7$ G01N33/53, G01N33/573, C07K16/40, C12Q1/48<br>C12N15/54, C12N9/12 |
| Documentation searched other than minimum documentation to the extent that such documents are included in the fields searched<br>Jitsuyo Shinan Koho 1922-1996 Toroku Jitsuyo Shinan Koho 1994-2000<br>Kokai Jitsuyo Shinan Koho 1971-2000 Jitsuyo Shinan Toroku Koho 1996-2000 |
| Electronic data base consulted during the international search (name of data base and, where practicable, search terms used)<br>JICST |

| | |
|---|---|
| C. DOCUMENTS CONSIDERED TO BE RELEVANT | |

| Category* | Citation of document, with indication, where appropriate, of the relevant passages | Relevant to claim No. |
|---|---|---|
| Y | JP, 10-48217, A (Igaku Seibutsugaku Kenyusho),<br>20 February, 1998 (20.02.98) (Family: none) | 1,2,5-8<br>9-16,18-20 |
| Y | JP, 4-504171, A (Euro-Diagnostics B.V.),<br>23 April, 1992 (23.04.92)<br>& WO, 90010234, A & AU, 5263190, A<br>& NL, 8900529, A & EP, 461174, A | 3,4,5,10-15<br>17-20 |
| Y | C. E. Canman et al., "Activation of the ATM Kinase by Ionizing Radiation and Phosphorylation of p53", SCIENCE, Vol.281, No.11, (1998), pp.1677-1679 | 1-20 |
| Y | S.Banin et al., "Enhanced Phosphorylation of p53 by ATM in Response to DNA Damage", SCIENCE, Vol.281, No.11, (1998), pp.1677-1679 | 1-20 |
| Y | SUSAN.P.LESS-MILLER et al., "Human DNA-Activated Protein Kinanase Phosphorylates Serines 15 and 37 in the Amino-Terminal Transactivation Domain of Human p53" MOLECULAR AND CELLULAR BIOLOGY, Nov, 1992, pp.5041-5049 | 1-20 |

| | |
|---|---|
| ☐ Further documents are listed in the continuation of Box C. | ☐ See patent family annex. |

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>15 August, 2000 (15.08.00) | Date of mailing of the international search report<br>05 September, 2000 (05.09.00) |
| Name and mailing address of the ISA/<br>Japanese Patent Office | Authorized officer |
| Facsimile No. | Telephone No. |

Form PCT/ISA/210 (second sheet) (July 1992)


## SEQUENCE LISTING

<110> Medical & Biological Laboratories co., Ltd.

<120> Method for measuring Protein Kinase Activity.

<130> M3-103PCT

<140>

<141>

<150> JP   1999-141187

<151> 1999-05-21

<160> 13

<170> PatentIn Ver. 2.0

<210> 1

<211> 398

<212> PRT

<213> Homo sapiens

<400> 1

Ser Leu Arg Arg Pro Lys Arg Ile Ile Ile Arg Gly His Asp Glu Arg
1               5                   10                  15

Glu His Pro Phe Leu Val Lys Gly Gly Glu Asp Leu Arg Gln Asp Gln
20            25            30

Arg Val Glu Gln Leu Phe Gln Val Met Asn Gly Ile Leu Ala Gln Asp
35            40            45

Ser Ala Cys Ser Gln Arg Ala Leu Gln Leu Arg Thr Tyr Ser Val Val
50            55            60

Pro Met Thr Ser Arg Leu Gly Leu Ile Glu Trp Leu Glu Asn Thr Val
65            70            75            80

Thr Leu Lys Asp Leu Leu Leu Asn Thr Met Ser Gln Glu Glu Lys Ala
85            90            95

Ala Tyr Leu Ser Asp Pro Arg Ala Pro Pro Cys Glu Tyr Lys Asp Trp
100            105            110

Leu Thr Lys Met Ser Gly Lys His Asp Val Gly Ala Tyr Met Leu Met
115            120            125

Tyr Lys Gly Ala Asn Arg Thr Glu Thr Val Thr Ser Phe Arg Lys Arg
130            135            140

Glu Ser Lys Val Pro Ala Asp Leu Leu Lys Arg Ala Phe Val Arg Met

145           150           155           160

Ser Thr Ser Pro Glu Ala Phe Leu Ala Leu Arg Ser His Phe Ala Ser

165           170           175

Ser His Ala Leu Ile Cys Ile Ser His Trp Ile Leu Gly Ile Gly Asp

180           185           190

Arg His Leu Asn Asn Phe Met Val Ala Met Glu Thr Gly Gly Val Ile

195           200           205

Gly Ile Asp Phe Gly His Ala Phe Gly Ser Ala Thr Gln Phe Leu Pro

210           215           220

Val Pro Glu Leu Met Pro Phe Arg Leu Thr Arg Gln Phe Ile Asn Leu

225           230           235           240

Met Leu Pro Met Lys Glu Thr Gly Leu Met Tyr Ser Ile Met Val His

245           250           255

Ala Leu Arg Ala Phe Arg Ser Asp Pro Gly Leu Leu Thr Asn Thr Met

260           265           270

Asp Val Phe Val Lys Glu Pro Ser Phe Asp Trp Lys Asn Phe Glu Gln

275           280           285

Lys Met Leu Lys Lys Gly Gly Ser Trp Ile Gln Glu Ile Asn Val Ala
290                 295                 300

Glu Lys Asn Trp Tyr Pro Arg Gln Lys Ile Cys Tyr Ala Lys Arg Lys
305                 310                 315                 320

Leu Ala Gly Ala Asn Pro Ala Val Ile Thr Cys Asp Glu Leu Leu Leu
                    325                 330                 335

Gly His Glu Lys Ala Pro Ala Phe Arg Asp Tyr Val Ala Val Ala Arg
                    340                 345                 350

Gly Ser Lys Asp His Asn Ile Arg Ala Gln Glu Pro Glu Ser Gly Leu
                    355                 360                 365

Ser Glu Glu Thr Gln Val Lys Cys Leu Met Asp Gln Ala Thr Asp Pro
370                 375                 380

Asn Ile Leu Gly Arg Thr Trp Glu Gly Trp Glu Pro Trp Met
385                 390                 395

<210> 2

<211> 362

<212> PRT

<213> Homo sapiens

<400> 2

Gly Val Asn Leu Pro Lys Ile Ile Asp Cys Val Gly Ser Asp Gly Lys
1               5                   10                  15

Glu Arg Arg Gln Leu Val Lys Gly Arg Asp Asp Leu Arg Gln Asp Ala
                20                  25                  30

Val Met Gln Gln Val Phe Gln Met Cys Asn Thr Leu Leu Gln Arg Asn
                35                  40                  45

Thr Glu Thr Arg Lys Arg Lys Leu Thr Ile Cys Thr Tyr Lys Val Val
                50                  55                  60

Pro Leu Ser Gln Arg Ser Gly Val Leu Glu Trp Cys Thr Gly Thr Val
65                  70                  75                  80

Pro Ile Gly Glu Phe Leu Val Asn Asn Glu Asp Gly Ala His Lys Arg
                85                  90                  95

Tyr Arg Pro Asn Asp Phe Ser Ala Phe Gln Cys Gln Lys Lys Met Met
                100                 105                 110

Glu Val Gln Lys Lys Ser Phe Glu Glu Lys Tyr Glu Val Phe Met Asp
                115                 120                 125

Val Cys Gln Asn Phe Gln Pro Val Phe Arg Tyr Phe Cys Met Glu Lys
  130    135    140

Phe Leu Asp Pro Ala Ile Trp Phe Glu Lys Arg Leu Ala Tyr Thr Arg
145    150    155    160

Ser Val Ala Thr Ser Ser Ile Val Gly Tyr Ile Leu Gly Leu Gly Asp
    165    170    175

Arg His Val Gln Asn Ile Leu Ile Asn Glu Gln Ser Ala Glu Leu Val
    180    185    190

His Ile Asp Leu Gly Val Ala Phe Glu Gln Gly Lys Ile Leu Pro Thr
   195    200    205

Pro Glu Thr Val Pro Phe Arg Leu Thr Arg Asp Ile Val Asp Gly Met
  210    215    220

Gly Ile Thr Gly Val Glu Gly Val Phe Arg Arg Cys Cys Glu Lys Thr
225    230    235    240

Met Glu Val Met Arg Asn Ser Gln Glu Thr Leu Leu Thr Ile Val Glu
    245    250    255

Val Leu Leu Tyr Asp Pro Leu Phe Asp Trp Thr Met Asn Pro Leu Lys
   260    265    270

Ala Leu Tyr Leu Gln Gln Arg Pro Glu Asp Glu Thr Glu Leu His Pro
                275                 280                 285

Thr Leu Asn Ala Asp Asp Gln Glu Cys Lys Arg Asn Leu Ser Asp Ile
                290                 295                 300

Asp Gln Ser Phe Asp Lys Val Ala Glu Arg Val Leu Met Arg Leu Gln
305                 310                 315                 320

Glu Lys Leu Lys Gly Val Glu Glu Gly Thr Val Leu Ser Val Gly Gly
                    325                 330                 335

Gln Val Asn Leu Leu Ile Gln Gln Ala Ile Asp Pro Lys Asn Leu Ser
                    340                 345                 350

Arg Leu Phe Pro Gly Trp Lys Ala Trp Val
                    355                 360

<210> 3

<211> 340

<212> PRT

<213> Homo sapiens

<400> 3

Ser Leu Gln Lys Pro Lys Lys Ile Ser Leu Lys Gly Ser Asp Gly Lys
1          5          10          15

Phe Tyr Ile Met Met Cys Lys Pro Lys Asp Asp Leu Arg Lys Asp Cys
20          25          30

Arg Leu Met Glu Phe Asn Ser Leu Ile Asn Lys Cys Leu Arg Lys Asp .
35          40          45

Ala Glu Ser Arg Arg Arg Glu Leu His Ile Arg Thr Tyr Ala Val Ile
50          55          60

Pro Leu Asn Asp Glu Cys Gly Ile Ile Glu Trp Val Asn Asn Thr Ala
65          70          75          80

Gly Leu Arg Pro Ile Leu Thr Lys Leu Tyr Lys Glu Lys Gly Val Tyr
85          90          95

Met Thr Gly Lys Glu Leu Arg Gln Cys Met Leu Pro Lys Ser Ala Ala
100          105          110

Leu Ser Glu Lys Leu Lys Val Phe Arg Glu Phe Leu Leu Pro Arg His
115          120          125

Pro Pro Ile Phe His Glu Trp Phe Leu Arg Thr Phe Pro Asp Pro Thr
130          135          140

Ser Trp Tyr Ser Ser Arg Ser Ala Tyr Cys Arg Ser Thr Ala Val Met
145             150             155             160

Ser Met Val Gly Tyr Ile Leu Gly Leu Gly Asp Arg His Gly Glu Asn
                165             170             175

Ile Leu Phe Asp Ser Leu Thr Gly Glu Cys Val His Val Asp Phe Asn
            180             185             190

Cys Leu Phe Asn Lys Gly Glu Thr Phe Glu Val Pro Glu Ile Val Pro
            195             200             205

Phe Arg Leu Thr His Asn Met Val Asn Gly Met Gly Pro Met Gly Thr
        210             215             220

Glu Gly Leu Phe Arg Arg Ala Cys Glu Val Thr Met Arg Leu Met Arg
225             230             235             240

Asp Gln Arg Glu Pro Leu Met Ser Val Leu Lys Thr Phe Leu His Asp
                245             250             255

Pro Leu Val Glu Trp Ser Lys Pro Val Lys Gly His Ser Lys Ala Pro
                260             265             270

Leu Asn Glu Thr Gly Glu Val Val Asn Glu Lys Ala Lys Thr His Val

275                     280                     285

Leu Asp Ile Glu Gln Arg Leu Gln Gly Val Ile Lys Thr Arg Asn Arg

290                     295                     300

Val Thr Gly Leu Pro Leu Ser Ile Glu Gly His Val His Tyr Leu Ile

305               310               315               320

Gln Glu Ala Thr Asp Glu Asn Leu Leu Cys Gln Met Tyr Leu Gly Trp

                325               330               335

Thr Pro Tyr Met

                340


<210> 4

<211> 14

<212> PRT

<213> Homo sapiens


<220>

<221> MOD_RES

<222> (7)

<223> PHOSPHORYLATION


<220>

<223> Description of Artificial Sequence:Artificially Synthesized Peptide.

<400> 4

Ser Val Glu Pro Pro Leu Ser Gln Glu Thr Phe Ser Asp Cys

1               5               10

<210> 5

<211> 14

<212> PRT

<213> Homo sapiens

<220>

<223> Description of Artificial Sequence:Artificially Synthesized Peptide.

<400> 5

Ser Val Glu Pro Pro Leu Ser Gln Glu Thr Phe Ser Asp Cys

1               5               10

<210> 6

<211> 14

<212> PRT

<213> Homo sapiens

<220>

<223> Description of Artificial Sequence:Artificially
Synthesized Peptide.


<220>

<221> MOD_RES

<222> (7)

<223> PHOSPHORYLATION


<400> 6

Pro Glu Asn Asn Val Leu Ser Pro Leu Pro Ser Gln Ala Cys

1               5                   10


<210> 7

<211> 14

<212> PRT

<213> Homo sapiens


<220>

<223> Description of Artificial Sequence:Artificially
Synthesized Peptide.


<400> 7

Pro Glu Asn Asn Val Leu Ser Pro Leu Pro Ser Gln Ala Cys

1                    5                         10

<210> 8

<211> 14

<212> PRT

<213> Homo sapiens


<220>

<223> Description of Artificial Sequence:Artificially

Synthesized Peptide.


<220>

<221> MOD_RES

<222> (7)

<223> PHOSPHORYLATION


<400> 8

Val Leu Ser Pro Leu Pro Ser Gln Ala Met Asp Asp Leu Cys

1                    5                         10


<210> 9

<211> 14

<212> PRT

<213> Homo sapiens

<220>

<223> Description of Artificial Sequence:Artificially Synthesized Peptide.

<400> 9

Val Leu Ser Pro Leu Pro Ser Gln Ala Met Asp Asp Leu Cys
1                   5                   10

<210> 10

<211> 30

<212> DNA

<213> Homo sapiens

<220>

<223> Description of Artificial Sequence:Artificially Synthesized Primer Sequence.

<400> 10

gcgaattcag gtgtaaattt accaaaaata                                    30

<210> 11

<211> 30

<212> DNA

<213> Homo sapiens

<220>

<223> Description of Artificial Sequence:Artificially

Synthesized Primer Sequence.

<400> 11

gcgctcgagc acccaagctt tccatcctgg                    30

<210> 12

<211> 30

<212> DNA

<213> Homo sapiens

<220>

<223> Description of Artificial Sequence:Artificially

Synthesized Primer Sequence.

<400> 12

gcgggatcct ctcttcagaa accaaagaag                    30

<210> 13

<211> 30

<212> DNA

<213> Homo sapiens

<220>

<223> Description of Artificial Sequence:Artificially

Synthesized Primer Sequence.


<400> 13

gcgctcgagc atatatggag tccaaccaag                                        30